Europäisches Patentamt

European Patent Office

Office européen des brevets

Publication number: **0 286 374**

**A2**

# EUROPEAN PATENT APPLICATION

Application number: 88303054.6

Int. Cl.⁴: **A61B 5/00 , G01N 21/00**

Date of filing: **06.04.88**

Priority: **07.04.87 US 35395**

Date of publication of application:
**12.10.88 Bulletin 88/41**

Designated Contracting States:
**AT BE CH DE ES FR GB GR IT LI LU NL SE**

Applicant: **QWIKCALL! CORPORATION**
**280 Brinkby Avenue**
**Reno Nevada 89509(US)**

Inventor: **Marzolf, Willis L.**
**2139 W. Elm Street Lodi**
**California 524(US)**
Inventor: **Snyder, Michael H.**
**2380 Piping Rock Drive Reno**
**Nevada 89502(US)**
Inventor: **Snyder, Michael J.**
**2380 Piping Rock Drive Reno**
**Nevada 89502(US)**
Inventor: **Fisher, Michael E. c/o Robert Dickey**
**Jr.**
**290 So. Arlington Ave. Reno**
**Nevada 89501(US)**

Representative: **Bedggood, Guy Stuart et al**
**Haseltine Lake & Co. Hazlitt House 28**
**Southampton Buildings Chancery Lane**
**London WC2A 1AT(GB)**

## Apparatus and method for sensing and preventing incontinent episodes.

The apparatus includes a probe (14) insertable in the colon with the probe having a sensor element (36) at the tip and a light transmissive element (62), such as a fiber optic cable, in optical communication with the sensor. The sensor reflects light transmitted to it and responds to the presence of fecal material to change the amount of reflected light. An incident light generator and a reflected light sensor respectively produce transmitted light and sense reflected light to generate an output proportional to the intensity of the reflected light. The probe is preferably two-piece having a disposable section for insertion; an inflatable cuff (38) is provided to block the colon; vent ports are provided to vent colonic gases during use; and a temperature sensor may be provided. The sensor element (36) may be a deformable reflective material or a lens. The method includes the steps of producing light at a source, transmitting a portion of the light to an optical element positioned in the colon, reflecting a different amount of light in response to the presence and absence of fecal material adjacent the optical element monitoring the intensity of the reflected light to generate an alarm indicative of the presence of fecal material.

FIG. 1

## APPARATUS AND METHOD FOR SENSING AND PREVENTING INCONTINENT EPISODES

The present invention relates to apparatus and methods for the detection, indication and prevention of incontinent episodes such as those often experienced by bed-ridden patients or others who have dysfunctional bowel control. The incontinent episode is both unpleasant and non-hygenic; the replacement of soiled bed linens, blankets and gowns compound the problems due to the loss of valuable nursing time and effort. Other problems with incontinent episodes include the excoriation of the patient's skin and the increased risk of fecal contamination of patients and nursing personnel. The present invention specifically addresses the problem of preventing incontinent episodes by detection and warning of a potential event.

The present invention may provide an apparatus and method for optically sensing the presence of fecal material in the human colon and preventing an incontinent episode.

Embodiments of the present invention may provide a method and apparatus for detecting and preventing an incontinent episode by means of an optical sensor that is inexpensive and disposable in order to maintain high sanitary conditions at a relatively low cost.

Further embodiments may provide the temporary prevention of an incontinent episode while automatically monitoring the colonic passage and providing a warning alarm of the presence of fecal material, both for bedridden and mobile patients.

According to one aspect, the present invention includes an apparatus that is adapted for optically monitoring the human colon to detect the presence of fecal material. This apparatus includes a rectal probe that is sized for insertion into the human colon through the anus; this probe includes a sensor element at its distal tip. A light source is provided and a light transmissive element, such as a fiber optic cable, passes this incident light to the probe and the sensor element. The sensor element reflects this incident light back through the light transmissive element so that the reflected light may be monitored by a light sensor which receives the reflected light from the light transmissive element and monitors the intensity of the reflected light and generates output proportional thereto. The sensor element is responsive to the present of fecal material to change the amount of reflected light. This is preferably accomplished by providing a deformable reflective element, but a relatively transparent, ellipsoidal lens may be used. An inflatable cuff extends around the probe to block the colon, and colonic gases are vented through the probe. A suitable control unit for this optical device is located remotely of the probe tip and is connected thereto by releaseable connectors.

A method according to the present invention includes the steps of producing light at a light source and transmitting at least a portion of the light as an incident light beam to a sensor element positioned in the colon. This method then includes the reflecting of a portion of the incident light beam by the sensor element in such a manner that the intensity of the reflected light beam changes in response to the presence and absence of fecal material contacting the sensor element. The intensity of the reflected light beam is then monitored to detect changes of the intensity, and an alarm signal is generated in response to a change in the intensity of the reflected light beam. The method may further include the steps of producing a reference light beam whereby the difference in intensity between the reference light beam and the reflected light beam is monitored. The method may also include the steps of physically blocking the colon to prevent the passage of fecal material, the venting of colonic gas to the body's exterior when the sensor element is in place and the simultaneous monitoring of colonic temperature.

Embodiments of the invention will now be described by way of example referring to the drawings:-

Figure 1 is a perspective view of the optical monitor for sensing and preventing incontinent episodes according to an embodiment of the present invention showing the control unit and rectal probe;

Figure 2 is a side view in elevation, partially broken away, of the rectal probe according to an embodiment of the present invention;

Figure 3 is a view in partial cross-section of the proximal end of a first portion of the colonic probe shown in Figure 3;

Figure 4 is a view in partial cross-section of the distal end of the first portion of the rectal probe shown in Figure 3;

Figure 5 is an enlarged view in cross-section of the distal end of the rectal probe showing the sensor element structure according to an embodiment of the present invention;

Figure 6 is an exploded view in perspective showing the construction of the sensor element at the distal end of the rectal probe according to an embodiment of the present invention;

Figure 7 is a top plan view, in representative format, of a control unit used with the rectal probe of the present invention;

Figure 8 is a cross-sectional view of a first alternate embodiment of the sensor element according to the present invention; and

Figure 9 is a cross-sectional view of a second alternate embodiment of the sensor element according to the present invention.

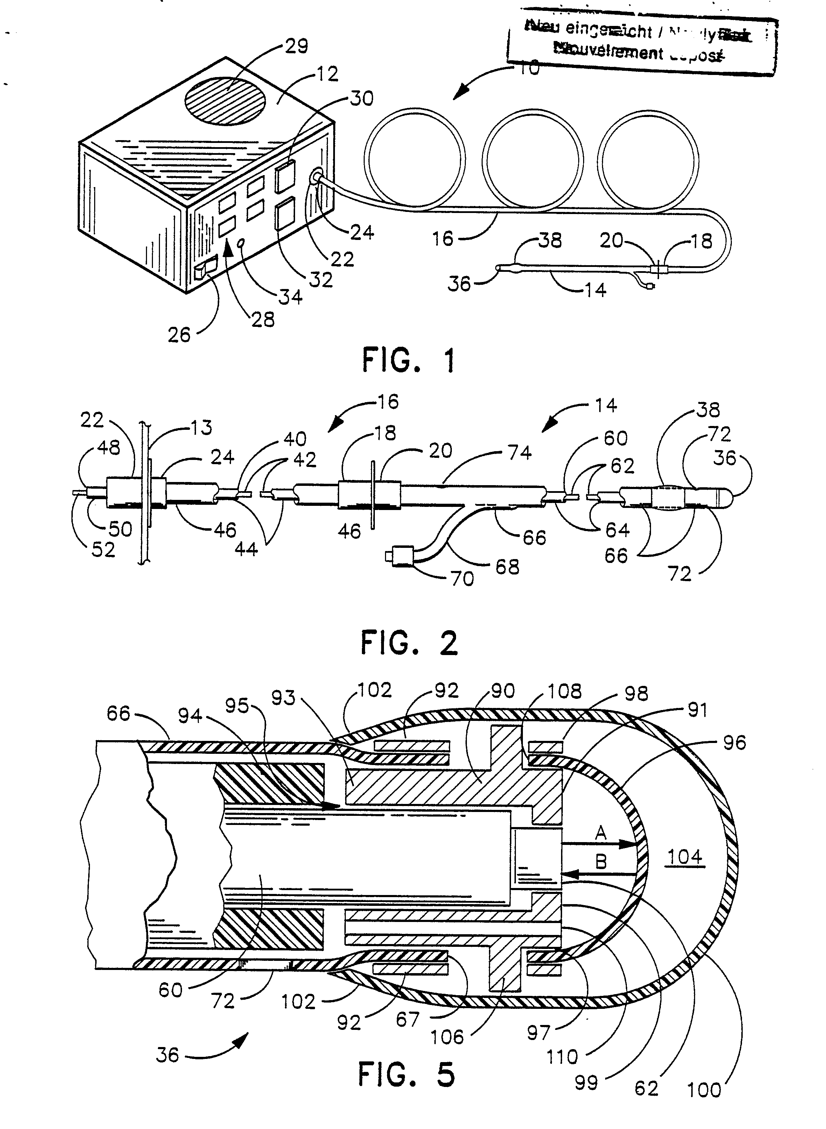

The present invention is directed to monitoring fecal mass in the colon, and specifically the human colon, and to the generation of an alarm signal detectable to the human senses that indicates such condition. According to an embodiment of the present invention, the incontinence monitoring apparatus 10, as is broadly shown in Figure 1, includes a rectal probe 14 which is connected to a control unit 12 by means of optical interconnect 16. More particularly, interconnect 16 is provided with a female connector 18 at its distal end which releaseably receives a mating male connector 20 located at the proximal end of rectal probe 14. A second female connector 22 is mounted in the housing of control unit 12 and mateably receives a corresponding male connector 24 located at the proximal end of interconnect 16.

Control unit 12 houses the signal generating and processing circuitry for monitor 10 and, as more thoroughly described below, includes a power switch 26 which activates control unit 12 which displays its operative condition on display panel 28. Control unit 12 may be placed in a calibration mode by switch 30 and, upon completion of self-calibration, may be placed in a monitoring mode by switch 32. A battery condition indicator light 34 is provided to notify the user that the battery supply for control unit 12 is in a low charge condition. Control unit 12 produces a reference light beam that travels through interconnect 16 and probe 14 so that it is presented to a sensor element 36 located at the distal end of probe 14. This distal portion of rectal probe 14 is adapted for insertion into the human colon through the anus and may be retained in position by means of an inflatable cuff 38. Upon the sensing of an impending incontinent episode, control unit 12 visually indicates an alarm state on display panel 28 and generates an audible alarm through speaker 29.

As is seen in Figure 2, female connector 22 is mounted in side panel 13 of control unit 12 and mateably receives male connector 24. Interconnect 16 comprises a fiber optic cable 40 that has a fiber optic element 42 surrounded by means of a protective sheath 44 as is known in the art. This fiber optic cable is then encased in a hollow flexible tube 46 that is formed of any suitable, medically approved rubberized material. Tube 46 is attached, at a proximal end, to male connector 24 so that a proximal end of fiber optic cable 40 extends into connector 24. At its distal end, tube 46 is attached to female connector 18 with fiber optic cable 40 being retained therein. Female connector 22 mounts and retains sheath 50 of a fiber optic cable 48. It should be appreciated that fiber optic element 52 of cable 48 is maintained in optical communication with fiber optic element 42 by means of connectors 22 and 24. Thus, any light that is directed through element 52 will be transmitted through connectors 22 and 24 to fiber optic element 42.

As is further shown in Figure 2, rectal probe 14 includes a fiber optic cable 60 which has a fiber optic element 62 encased in a protective sheath 64. Cable 60 is received in a flexible tube 66 constructed of a flexible rubberized material such as latex as is commonly used for existing rectal catheters. A proximal end of tube 66 is mounted in male connector 20 so that fiber optic cable 60 extends through connector 22. Thus, fiber optic element is in optical communication with fiber optic element 42 of fiber optic cable 40. Connectors 18 and 20 secure the distal end of interconnector 16 to the proximal end of rectal probe 14 in a releaseable manner that still optically transmits light between fiber optic elements 42 and 62. Rectal probe 14 includes an inflatable cuff 38 located in proximity to the distal end of probe 14. Inflatable cuff 38 is a balloon-like structure that may be inflated by means of fill tube 68 which is provided with a fill valve 70. Valve 70 is of a standard shut off type that receives the outlet nub of a hypodermic syringe which injects a measured amount of air.

The distal end of rectal probe 14 is provided with a novel sensor element 36, and tube 66 is provided with a pair of vent ports 72 formed in the end of tube 66 at a location between cuff 38 and sensor 36. A vent port 74 is located at the proximal end of probe 14. Vents 72 and 74 allow the passage of colonic gases through tube 66 while cuff 38 is inflated to block the elimination of rectal material from the colon.

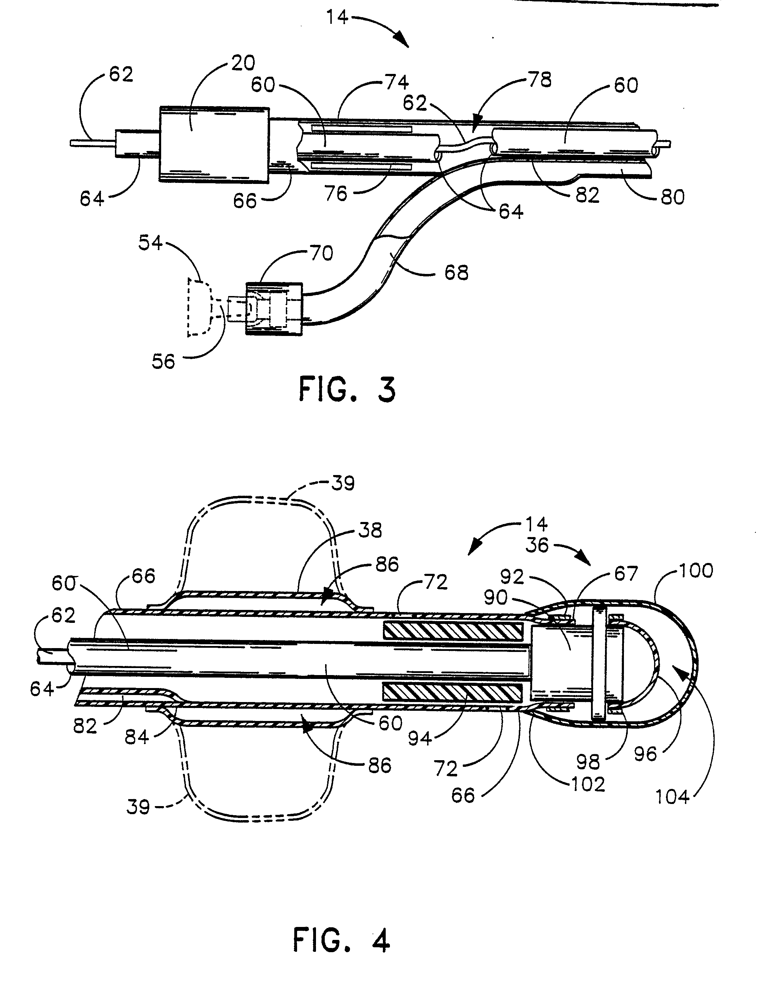

The construction of the proximal and distal ends of rectal probe 14 are shown in greater detail in Figures 3 and 4. As is shown in Figure 3, the proximal end of rectal probe 14 is shown with fiber optic cable 60 being axially received in flexible tube 66 which is, in turn, secured to connector 20 so that sheath 64 and fiber optic element 62 protrude slightly from connector 20. A colonic gas filter 76 is in the form of a hollow cylindrical member which surrounds fiber optic cable 60 at a location adjacent vent port 74. As is seen in Figure 3, tube 66 is separated into a first axial chamber 78 and a fill passageway 80 by means of interior wall 82. Fill tube 68 is in fluid communication with fill passageway 80. As is shown in phantom, a hypodermic syringe 54 has a nub 56 that may be received in automatic shut-off valve 70 so that air may be injected through valve 70 by means of operation of the plunger of syringe 54. This injected air then passes through fill tube 68 and into fill passageway 80 so that it may be transferred to inflatable cuff 38. As is shown in Figure 4, fill passageway 80 terminates in a distal opening 84 which is in fluid communication with torroidal chamber 86 of inflatable cuff 38. Upon the injection of air, cuff 38 expands, in a balloon-like manner, to the inflated position shown in phantom at 39 in Figure 4.

The construction of the distal end of rectal tube 14 is shown in greater detail in Figure 4. Here, sensor element 36 includes a housing element 90 which is mateably received in distal end 67 of tube 66. A constriction band 92, which may be of heat shrinkable material, extends around the circumference of tube 66 adjacent distal end 67 and is operative to secure tube 66 to housing element 90. Housing element 90 receives and mounts fiber optic cable 60 which is telescopically received through a hollow cylindrical colonic filter 94 which is positioned around cable 60 at vents 72. Housing element 90 also mounts a deformable reflective strip 96 by means of a spring clip 98 which extends partially around the circumference of the end of housing element 90. The distal end of housing element 90 is then enclosed by means of a flexible latex tip 100 that is adhered at edge 102 to the distal end 67 of tube 66. Thus, tip 100 acts as a flexible end cap for sensor element 36 and defines a sensor chamber 104 around strip 96.

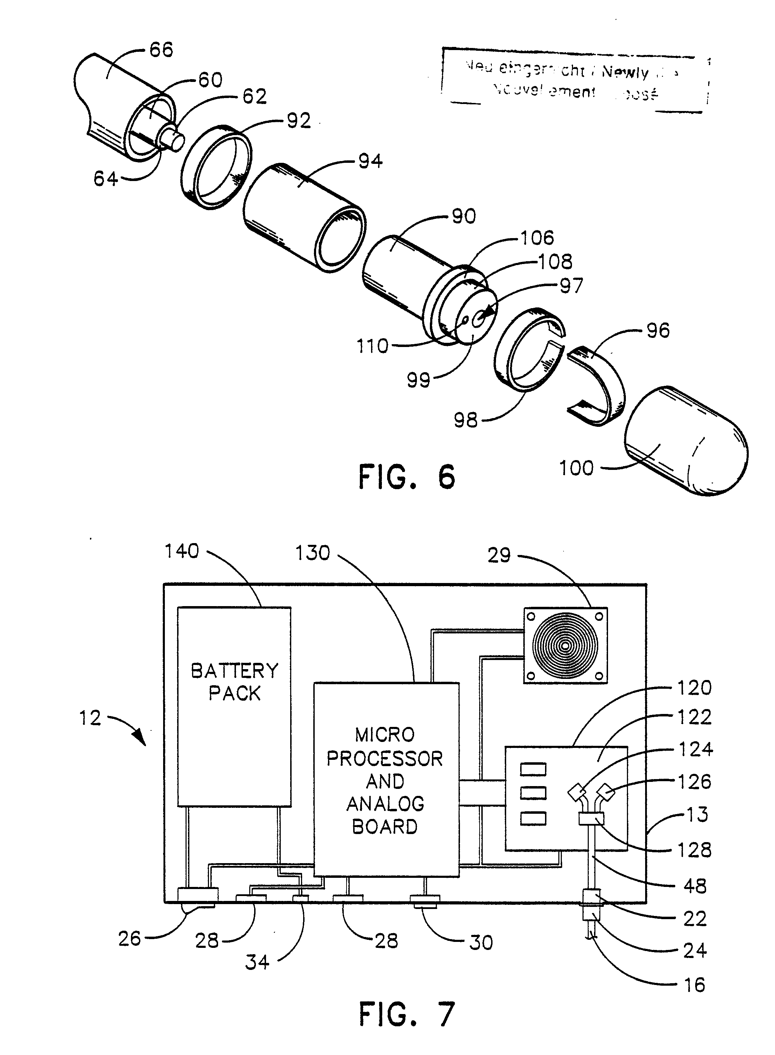

The construction of the sensor element 36 may now be more readily understood with reference to Figures 5 and 6. Here, housing element 90 is generally cup-shaped in configuration and has a flat endwall 91 and a surrounding cylindrical sidewall 93 which defines a cavity 95 therein. Cavity 95 mateably receives the distal end of fiber optic cable 60 with cable 60 being retained in cavity 95 by any suitable adhesive. An axial opening 97 is formed in end wall 91 with this opening 97 receiving fiber optic element 62 so that the end of fiber optic element 62 is relatively flush with end surface 99 of end wall 91. A radially outwardly projecting flange 106 extends around the circumference of sidewall 93 to define a flat shoulder 108.

Reflective strip 96 is formed as an arcuate band of reflective material, such as mylar, which is also resilient. Strip 96 arcuately extends from opposite diametric locations on shoulder 108 so that strip 96 forms an arch across end surface 99. The opposite edges of strip 96 abut shoulder 108 and are retained against shoulder 108 by means of a C-shaped spring clip 98. It may thus be appreciated that incident light, represented by arrow A may be reflected as reflected light indicated at arrow B. Thus, incident light from optical element 62 is incident on the inner surface of strip 96 and is reflected back to optical element 62 as a reflected light beam. This reflected light beam will then be transmitted back to control unit 12 by means of optical elements 62 and 42.

As is noted above, tube 66 is mounted to housing 90 by means of a constriction element 92 which is preferably a heat shrinkable band that extends around the circumference of tube 66, adjacent distal end 67, Band 92 may be heat shrunk onto tub 66 to collapse it on sidewall 93 of housing element 90. Protective tip 100 is then formed around element 90 and strip 96 as a protective boot, and this tip may be conveniently formed of a thin Latex material that is bonded at edge 102 to tube 66. Thus, tip 100 defines a sensor chamber 100 which would normally define a sealed environment. In order to avoid unwanted inflation or deflation of tip 100 by means of the pressure of gases in chamber 104, chamber 104 is vented by means of a vent passageway 110 that is formed longitudinally through sidewall 93. Thus, the pressure of chamber 104 is equalized with the colonic environment since chamber 104 is in fluid communication with colonic gases entering through vents 72.

Before discussing the operation of the incontinence monitor apparatus 10 according to the present invention, a functional understanding of control unit 12 is necessary. Control unit 12 is shown, in representative format, in Figure 7. It should be appreciated that different control units 12 are within the scope of the present invention so that control unit 12 is described for purposes of explanation and not limitation. As is shown in Figure 7, then, control unit 12 broadly includes a sensor board 120, a microprocessor board 130, a battery pack 140 and audio alarm or speaker 29. With reference to sensor board 20, it may be appreciated that light producing and sensing unit 122 includes a light emitting diode 124 that is coupled to fiber optic cable 48. A photo cell 126 is also optically coupled to optical cable 48, all by optical connector 128. Accordingly, light produced as incident light by LED 124 is passed through fiber optic cable 48 to fiber optic cable 40 of interconnect 16. Likewise, reflected light passing back through fiber optic cable 40 of interconnect 16 is received by photo cell 126.

Microprocessor and analog board 130 contain the processing circuitry which monitors the intensity of the incident light and the reflected light and compares the relative distance between the outputs of LED 124 and the photo cell 126 which corresponds to the relative intensities of the incident and reflected light. Micro processor 130 monitors electrical current and measures the magnitude of the difference between these outputs. To this end, the microprocessor only periodically samples the incident light and the reference reflected light received from sensor 36 to reduce the duty cycle, thus conserving the stores energy of battery pack 140. This sampling may, for example, be set to occur every 5 to 10 seconds. At such time that the difference between the two outputs exceeds a preselected value, microprocessor 130 creates a visual alarm on display panel 28 and an audible alarm through speaker 29. It should further be appreciated that microprocessor 130 is self-calibrating so that, when first activated with a patient, a calibration switch is activated so that microprocessor 130 automatically sets the selected magnitude difference between intensities of incident and reflected light. Thus, after a desired interval wherein microprocessor 30 measures the difference between the outputs, switch 32 may be activated to place control unit 12 in a monitoring mode. When the self-calibrated difference between the outputs exceeds a set threshold, then, microprocessor 130 activates the alarms. Control unit 12, as is readily understood. is powered by a battery pack 140 and is activated by means of switch 26. Monitor light 34 activates when battery pack 140 reaches a low charge condition so that it may be recharged or replaced.

The use of the present apparatus may now be more fully appreciated. In operation, interconnect 16 is first connected to control unit 12 by means of connec tors 22 and 24. Rectal probe 14, which is constructed to be disposable, is then connected to interconnect 16 by means of connectors 18 and 20. The distal end of probe 14 is then lubricated and inserted into the human colon through the anus so that inflatable cuff 38 is located inside of the colon. Cuff 38 is inflated by means of hypodermic syringe 54, and syringe 54 is removed; shut off valve 70 maintains cuff 38 in the inflated condition. Control unit 12 is activated which causes light to be generated by LED 124 which passes through the fiber optic cables to be presented as incident light onto the inner surface of strip 96. A reference reflected light is returned through the fiber optic cable and is monitored by the light sensing means in the form of photo cell 126. Switch 30 is then acti vated to place microprocessor 130 in the self-calibration mode wherein the difference in intensities of the incident light and the reference reflected light is sampled. After a selected interval of time, switch 32 is activated to place control unit 12 in a monitoring mode; control unit 12 now periodically samples the reflected and incident light intensities. At such time that fecal material moves down the colon and contacts sensor 36, tip 100 is forced to collapse and, correspon dingly, deforms the shape of strip 96. When strip 96 becomes deformed, the reference reflected light is modified as modified reflected light which changes the output of photo cell 126. Microprocessor 130 reacts to this change in signals which. when it changes from the preselected magnitude by a threshold percentage, causes the sounding of an audible alarm and a visual display of an impending bowel movement.

From the foregoing, it should be appreciated that the present invention may be used by bedridden patients and by ambulatory patients by making control unit 12 portable. Inflatable cuff 38 blocks the undesirable elimination of fecal material until such time that rectal probe 14 may be disconnected from interconnect 16 and either the bedridden patient be personally attended to or the mobile patient have to reach toilet facilities. At such time, syringe 54 is again inserted into valve 70 to allow the collapse of cuff 38, and rectal probe 14 is removed from the colon. The fecal material may then be eliminated in a controlled manner which avoids the unwanted soiling of bed linens, clothes and the like. Rectal probe 14 is disposed of and a sterile probe inserted to prevent the next incontinent episode.

Accordingly, it should be appreciated that the method of an embodiment of the present invention comprises the broad steps of producing light at a light source, transmitting at least a first portion of the light as an incident light beam through an optical element positioned in the human colon, reflecting a portion of the incident light beam as reflected light in such manner that the intensity of the reflected light beam changes in response to the presence and absence of fecal material contacting the sensor element. The intensity of the reflected light is monitored to produce first output corresponding to the intensity and an alarm signal is generated in response to a change in the first output.

This method may further include the steps of transmitting a second portion of the light as a reference light beam so that the intensity of the reference beam is monitored to produce second output, and the first and second outputs are compared to generate an alarm signal when the difference in intensities of the two outputs exceeds a preselected threshold. The values of the first and second outputs may be periodically checked. The method may include the further steps of physically blocking the colon during the monitoring process to prevent the unwanted discharge of fecal material while the sensor element is in the colon, and the venting of colonic gases to the environment while the colon is physically blocked.

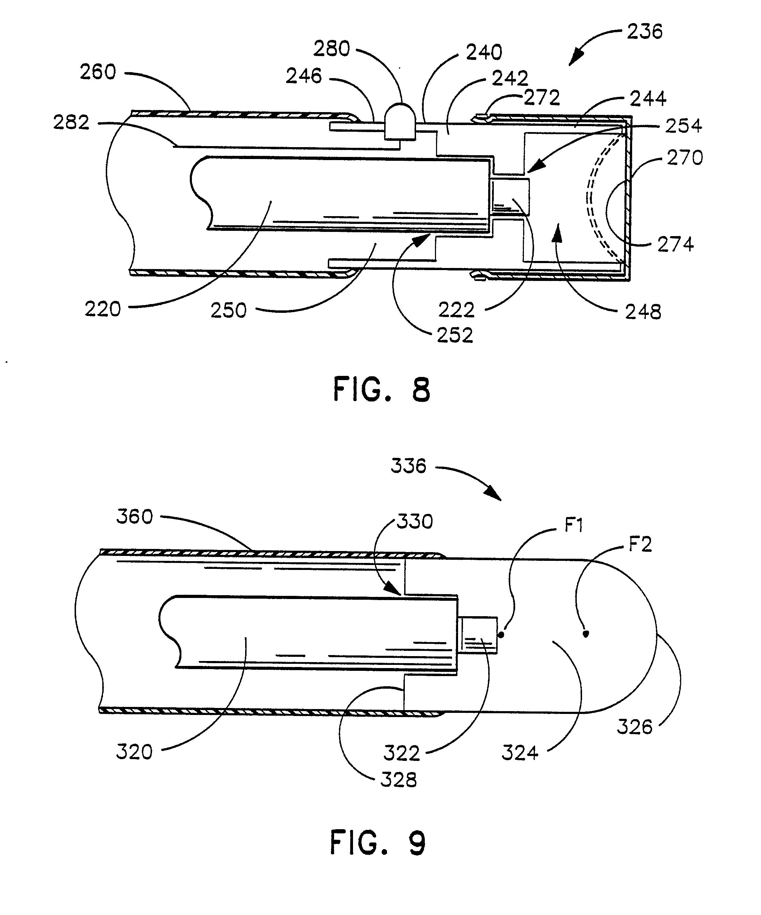

Figures 8 and 9 show alternative embodiments of the preferred invention. In figure 8, a sensor element 236 is shown and comprises a double cup-shaped housing element 240 which has a central portion 242 and oppositely projecting cylindrical sidewalls 244 and 246 which respectively define cavities 248 and 250. A flexible tube 260 is adhered to the outer surface of sidewall 246 and a fiber optic cable 220 actually extends through tube 260 and is received in a cable cavity 252 formed in central portion 242. A smaller bore 254 communicates with cavity 252 and receives fiber optic element 222 therethrough. The distal end of sensor 236 is enclosed by means of a flexible end cap 270 which extends along the outer surface of sidewall 242 and is secured thereto in any convenient manner such as by compression band 272. End cap 270 includes an internally reflective flexible surface 274 which reflects incident light from fiber optic element 222 in a manner similar to that with respect to band 96 described in the preferred embodiment of the present invention. A temperature sensor 280 may also be provided in this alternate embodiment with sensor 280 being mounted through sidewall 246 and connected back to control unit 12 by means of a wire 282. Thus, the preferred embodiment of the present invention contemplates the use of a temperature sensing element to monitor the colonic temperature and the method according to the present invention further contemplates the steps of monitoring and displaying the colonic temperature by control unit 12.

In the second alternate embodiment, shown in Figure 9, sensor element 336 operates on the principle of internal reflection. In this embodiment, a lens 324 is formed out of any convenient transparent material. The distal end 326 has a forward face that is ellipsoidal in shape having two focii $F_1$ and $F_2$. The proximal end 328 of lens 324 is mateably received in flexible tube 360 and is affixed therein by means of any suitable adhesive. A cavity 330 is formed in the proximal end of lens 324 and a distal end of fiber optic 320 is mounted therein so that fiber optic element 322 projects into the interior of lens 324 to terminate at focal point $F_1$.

In operation, the ellipsoidal shape of lens 324 causes internal reflection of a substantial portion of light emitted from fiber optic element 322. When fecal material contacts end 326, however, the interface characteristics change so that more light is absorbed at end 326 which reduces the amount of internally reflected light. This correspondingly changes the intensity of the reflected light beam so that control unit 12 will activate the alarm.

## Claims

1. Apparatus adapted for sensing the presence of fecal material in the human colon, comprising a probe sized for insertion into the human colon through the anus, said probe having a light transmissive element and a sensor element located at a distal end of said probe, light source means for generating incident light whereby said incident light is transmitted through the light transmissive element to said sensor element, said sensor element operative to internally reflect a portion of said light as reflected light that is transmitted back through said light transmissive element and whereby said sensor element responds to the presence of fecal material therearound to change the amount of incident light internally reflected thereby, and light sensor means for receiving reflected light from said light transmissive element and generating first output proportional to the intensity of the reflected light.

2. Apparatus according to claim 1 including monitor means for monitoring said first output and for generating an alarm signal in response to a change in said first output.

3. Apparatus according to claim 2 wherein said light sensor means is adapted for further monitoring said incident light and generating second output proportional to the intensity of the incident light, said monitor means including comparator means for comparing said first and second outputs, said alarm signal being generated when a difference in magnitude between the first and second outputs exceed a selected value.

4. Apparatus according to claim 3 wherein said monitor means comprises microprocessor means, said microprocessor means having sampling circuitry operative to periodically sample said first and second outputs.

5. Apparatus according to any preceding claim including a control unit housing said light source means and said light sensor means remotely from said probe, an optical cable optically coupling said light source means, said light sensor means and said light transmissive element in a releaseable manner whereby light generated by said light source means is transmitted through said optical cable and said light transmissive element as incident light and is reflected by said sensor element back through said light transmissive element and said optical cable as reflected light.

6. Apparatus according to any preceding claim wherein said probe is formed as a generally hollow tubular member having said sensor element on one end thereof and including at least one port operative to permit colonic gases to pass into the interior thereof, and including an inflatable cuff surrounding the probe, said port located between the cuff and said sensor element, and conduit means in communication with said cuff for allowing inflation of the cuff after the probe has been inserted in the colon to prevent passage of fecal material therearound.

7. Apparatus according to claim 6 including a filter element in said probe and associated with said port for filtering the colonic gases into the probe.

8. Apparatus according to any preceding claim including temperature measuring means on said probe for measuring the temperature of the colon when the probe is placed therein.

9. Apparatus according to any preceding claim wherein said sensor element includes a deformable element having a normal state operative to reflect incident light as reference reflected light, and moveable in response to pressure of fecal material into a deformed state operative to reflect incident light as modified reflected light so that the amount of light reflected back through the light transmissive element changes between the reference reflected light and modified reflected light.

10. Apparatus according to claim 9 wherein the light transmissive element is a fiber optic cable, having an inner fiber optic element and an outer sheath, said sensor element including a cup-shaped housing element having an end wall and a surrounding sidewall defining a cable cavity mateably receiving and securing a distal end of said fiber optic cable said endwall having a port therein sized to receive the fiber optic element so that the end of said fiber optic element is relatively flush with an outer surface of said endwall, said deformable element being an arcuate band of reflective resilient material mounted as an arch over the outer surface of said endwall.

11. Apparatus according to claim 10 including a protective flexible tip extending and enclosing the cup-shaped housing element.

12. Apparatus according to claim 9 wherein said sensor element is defined by a body member having a cavity formed in its distal end and a flexible diaphragm extending over and sealing said cavity and defining said distortable element, said diaphragm having a reflective surface facing said light transmissive element.

13. Apparatus according to any one of claims 1 to 9 wherein said sensor element is defined by a relatively transparent lens.

14. Apparatus according to claim 13 wherein said lens has a distal ellipsoidal surface, said light transmissive element terminating in said lens at a first focal point with the other focal point of the ellipse being located between said first focal point and said ellipsoidal surface.

15. Apparatus according to any preceding claim wherein said probe is formed as a first probe portion having a first portion leading end adapted for insertion into the colon and a first probe portion trailing end protruding from the anus, and a second probe portion having second probe portion leading end releaseably secureable in optical communication with the first probe portion trailing end, and a releaseable connector means for securing the first probe portion trailing end to the second probe portion leading end.

16. A rectal probe adapted for sensing the presence of fecal material in the human colon by transmitting an incident light beam and a reflected light beam, comprising:

a fiber optic cable operative to receive and transmit said incident light beam from a light source; and

a sensor element located at a distal end of said fiber optic cable and operative to reflect said reflected light beam back through said fiber optic cable to a light detector, said sensor element having response means for responding to contact between the sensor element and fecal material to change the amount of light reflected as said reflected light beam.

17. A rectal probe according to claim 16 including a generally hollow tubular member receiving said fiber optic cable having said sensor element on one end thereof and including at least one port operative to permit colonic gases to pass into the interior thereof, said tubular member having an inflatable cuff surrounding a portion thereof proximate said sensor element, said port located between the cuff and said sensor element, and conduit means in communication with said cuff for allowing inflation of the cuff after the probe has been inserted in the colon to prevent passage of fecal material therearound.

18. A rectal probe according to claim 17 wherein said sensor element includes a deformable element having a normal state operative to reflect incident light as reference reflected light, said deformable element moveable in response to pressure of fecal material into a deformed state operative to reflect incident light as modified reflected light so that the amount of light reflected back through the fiber optic cable changes between the reference reflected light and modified reflected light.

19. A method for detecting the presence of fecal material in the human colon to prevent incontinent episodes, comprising the steps of:

producing a light at a light source;

transmitting at least a first portion of said light as an incident light beam to an optical element positioned in the colon;

reflecting a portion of the incident light beam by the optical element as a reflected light beam in such a manner that the intensity of the reflected light beam changes in response to the presence and absence of fecal material adjacent the optical element;

monitoring the intensity of the reflected light beam and producing first output corresponding thereto; and

generating an alarm signal in response to a change in the first output.

20. The method according to claim 19 including the steps of transmitting a second portion of the light produced by the light source as a reference light beam, monitoring the intensity of the reference beam and producing second output corresponding thereto, comparing the first and second outputs to generate said alarm signal when the difference in intensities exceeds a preselected threshold.

21. The method according to claim 20 including the step of periodically sampling each of said first and second outputs.

22. The method according to claim 19 including the step of physically blocking the colon to prevent discharge of fecal material while the optical element is in the colon.

23. The method according to claim 22 including the step of venting colonic gases to the environment external of the colon concurrently with the step of physically blocking the colon.

0 286 374

FIG. 1

FIG. 2

FIG. 5

FIG. 3

FIG. 4

FIG. 6

FIG. 7

FIG. 8

FIG. 9